# EUROPÄISCHE PATENTSCHRIFT

(11) **EP 3 587 340 B1**
(45) Veröffentlichungstag und Bekanntmachung des Hinweises auf die Patenterteilung: **21.07.2021**
(21) Anmeldenummer: 19178401.6
(22) Anmeldetag: 05.06.2019
(51) Int. Cl.: B67B 1/03, B67B 3/00

(54) **VORRICHTUNG ZUM BEHANDELN VON BEHÄLTERVERSCHLÜSSEN**
DEVICE FOR TREATING CONTAINER CLOSURES
DISPOSITIF DE MANIPULATION DES FERMETURES DE RÉCIPIENTS

(30) Priorität: 05.06.2018 DE 102018113291
(43) Veröffentlichungstag der Anmeldung: 01.01.2020
(60) Teilanmeldung: 21171899.4
(73) Patentinhaber: KRONES AG, 93073 Neutraubling (DE)
(72) Erfinder: Mueller, Holger, 93073 Neutraubling (DE); Soellner, Juergen, 93073 Neutraubling (DE)
(74) Vertreter: Nordmeyer, Philipp Werner

(56) Entgegenhaltungen:
- EP-A1- 0 794 903
- DE-A1-102016 122 462
- JP-A- H0 858 965
- US-A1- 2016 207 717
- US-A1- 2018 074 086
- US-B2- 9 061 873

## Beschreibung

### Technisches Gebiet

Die vorliegende Erfindung betrifft eine Vorrichtung zum Behandeln von Behälterverschlüssen, insbesondere zum Sterilisieren von Behälterverschlüssen zum Verschließen von Behältern in einer Getränkeabfüllanlage.

### Stand der Technik

In Getränkeabfüllanlagen ist es allgemein bekannt, Behälterverschlüsse zu behandeln, bevor sie auf befüllte Behälter aufgebracht werden, um diese zu verschließen. Insbesondere werden die Behälterverschlüsse sterilisiert, damit durch die Behälterverschlüsse keine Keime oder anderweitige Verunreinigungen in die dann verschossenen Behälter und insbesondere nicht in das in den Behältern aufgenommene Füllgut gelangen können.

Bei herkömmlichen Anlagen ist es bekannt, die Behälterverschlüsse mittels eines Kaskadenförderers bezüglich ihrer räumlichen Orientierung zu sortieren und zugleich in die Höhe zu fördern und dann einer Behandlungskammer zum Sterilisieren der Behälterverschlüsse zuzuführen. Die Behandlungskammer kann beispielsweise oberhalb eines Reinraums angeordnet sein, um ein Zuführen zu einem in dem Reinraum angeordneten Verschließer allein durch die Schwerkraft zu ermöglichen.

Hierbei ist es beispielsweise bekannt, in aseptischen Getränkeabfüllanlagen Behälterverschlussdesinfektionssysteme einzusetzen, mittels derer die auf die dann befüllten Behälter aufzusetzenden Behälterverschlüsse sterilisiert beziehungsweise desinfiziert werden. In der Regel werden in derartigen Behandlungskammern Peressigsäure und/oder Wasserstoffperoxid in Dampfform zum Sterilisieren der Behälterverschlüsse verwendet. Diese Behälterverschlusssterilisierungssysteme sind beispielsweise mit einer eigenen Führungsrinne versehen, in welcher die zu sterilisierenden beziehungsweise zu desinfizierenden Behälterverschlüsse aufgrund der Schwerkraft entlang gleiten und dabei mit dem Sterilisierungsmittel oder Desinfektionsmittel beaufschlagt werden.

Diese Art von Vorrichtungen zum Behandeln von Behälterverschlüssen baut hoch und erfordert daher hohe Hallenhöhen von bis zu 10 m Deckenhöhe. Um den erforderlichen Durchsatz an Behälterverschlüssen zu erzielen, liegt stets eine Vielzahl von Behälterverschlüssen zeitgleich in der Führungsrinne in der Behandlungskammer vor. Entsprechend kann es durch den Staudruck der durch die Führungsrinne geführten Behälterverschlüsse zu einem Verkeilen oder Verklemmen der Behälterverschlüsse in der Führungsrinne kommen.

Die Führungsrinne muss dabei auf den jeweiligen Behälterverschluss-Typ ausgelegt sein, um eine problemlose und lagerichtige Führung der Behälterverschlüsse durch die Behandlungskammer hindurch und zu dem Verschließer hin zu erreichen. Bei einem Wechsel des Typs der verwendeten Behälterverschlüsse muss dementsprechend die Führungsrinne ausgewechselt werden, oder es werden unterschiedliche Führungsrinnen parallel zueinander in der Behandlungskammer vorgesehen, zwischen denen dann umgeschaltet werden kann.

Die Führungsrinne kann am Ende der Behandlungskammer, in welcher die Behandlung der Behälterverschlüsse erfolgt, einen Stopper aufweisen, welcher verhindert, dass ein Behälterverschluss weiter durch die Führungsrinne rutschen kann. Dies ist notwendig, um sicherzustellen, dass jeder Behälterverschluss - auch der erste, der noch nicht auf einer Säule von Behälterverschlüssen aufsitzt - die erforderliche Behandlungszeit in der Behandlungskammer verbracht hat.

Aufgrund des aggressiven Milieus innerhalb der Behandlungskammer, insbesondere hervorgerufen durch die darin vorherrschende Temperatur und die Konzentration des Sterilisationsmittels, muss die Verweilzeit der Behälterverschlüsse in der Behandlungskammer relativ genau eingehalten werden.

Bei einem zu kurzen Verweilen in der Behandlungskammer besteht die Gefahr, dass die Behälterverschlüsse nicht ordnungsgemäß sterilisiert werden. Behälterverschlüsse, die hingegen zu lange in der Behandlungskammer verweilen, beispielsweise aufgrund eines Verklemmens der Behälterverschlüsse in der Führungsrinne oder aufgrund eines Anlagenstopps, müssen hingegen verworfen werden und können nicht zum Verschließen eines Behälters verwendet werden, da beispielsweise das Material der Behälterverschlüsse zu lange thermisch behandelt wurde oder durch das Behandlungsmittel zu stark angegriffen wurde. Die Behälterverschlüsse können sich in der Führungsrinne auch aufgrund der Temperatur und wegen des Staudrucks verformen, wenn sie zulange in der Behandlungskammer verweilen.

Da die Führungsrinne schwerkraftbedingt nur einseitig fördern kann, müssen die nicht mehr verwendbaren, weil beispielsweise überbehandelten, Behälterverschlüsse nach unten hin ausgeschleust werden. In der Regel landen sie dann am Boden des Isolators der Getränkeabfüllanlage. Ein Öffnen und Ausräumen dieser Behälterverschlüsse hat jedoch eine Verletzung des Reinraumzustands des Isolators zur Folge, wodurch eine Zwischensterilisation erforderlich ist. Es wird entsprechend versucht, dies zu vermeiden.

Die US 2018/0074086 A1 beschreibt ein System und Verfahren zum Befördern von mit Behältern beladenen Transportmitteln, um unterschiedliche Endprodukte zu fertigen. Die US 2016/0207717 A1 beschreibt eine Vorrichtung und ein Verfahren zum Bewegen von Transportelementen in einer Behälterbehandlungsanlage. Die DE 10 2016 122 462 A1 beschreibt eine Vorrichtung zum Sortieren und Fördern von Behälterverschlüssen. Die US 9,061,873 B2 beschreibt eine Vorrichtung und ein Verfahren zum Herstellen von Kunststoffbehältnissen. Die WO 96/18541 A1 beschreibt ein System und Verfahren zum sterilen Verpacken von Getränken.

### Darstellung der Erfindung

Ausgehend von dem bekannten Stand der Technik ist es eine Aufgabe der vorliegenden Erfindung, eine verbesserte Vorrichtung zum Behandeln von Behälterverschlüssen, insbesondere zum Sterilisieren von Behälterverschlüssen für Behälter in einer Getränkeabfüllanlage, bereitzustellen. Die Aufgabe wird durch eine Vorrichtung zum Behandeln von Behälterverschlüssen, bevorzugt zum Sterilisieren von Behälterverschlüssen für Behälter in einer Getränkeabfüllanlage, mit den Merkmalen des Anspruchs 1 gelöst. Vorteilhafte Weiterbildungen ergeben sich aus den Unteransprüchen, der Beschreibung und den beigefügten Figuren.

Entsprechend wird eine Vorrichtung zum Behandeln von Behälterverschlüssen, bevorzugt zum Sterilisieren von Behälterverschlüssen zum Verschließen von Behältern in einer Getränkeabfüllanlage, vorgeschlagen, umfassend eine Behandlungskammer zum Behandeln von Behälterverschlüssen, und eine Transportvorrichtung zum Transportieren von Behälterverschlüssen durch die Behandlungskammer. Erfindungsgemäß umfasst die Transportvorrichtung einen Linearantrieb, wobei der Linearantrieb in einer Förderrichtung und einer Gegenförderrichtung betreibbar ist.

Dadurch, dass die Transportvorrichtung einen Linearantrieb umfasst, kann die Bewegung eines Behälterverschlusses oder mehrerer Behälterverschlüsse durch die Behandlungskammer individuell und variabel angepasst werden. Folglich ist es möglich, die Behandlungszeit der Behälterverschlüsse in der Behandlungskammer zeitlich zu variieren beziehungsweise an den jeweiligen Typ der Behälterverschlüsse anzupassen. So kann eine Kammergröße der Behandlungskammer für verschiedene Behälterverschluss-Typen verwendet werden. Ebenso ist es nicht notwendig, verschiedene Führungsrinnen, welche in einer einzigen Behandlungskammer verschiedene Durchführwege für die verschiedenen Behälterverschlüsse und auch verschiedene Behandlungszeiten bereitstellen, zu verwenden.

Weiterhin ist es dadurch, dass die Transportvorrichtung einen Linearantrieb umfasst, nicht mehr notwendig, die zu behandelnden Behälterverschlüsse zuerst über einen Massenförderer zu heben und anschließend durch eine Führungsrinne schwerkraftbedingt herabgleiten zu lassen. Vielmehr sind durch den Linearantrieb auch horizontale Bewegungen und vertikale Bewegungen mit und entgegen der Schwerkraft möglich. Die Behandlung der Behälterverschlüsse kann somit auch bodennah erfolgen. Es ist folglich nicht notwendig, Platz beziehungsweise technische Maßnahmen für die Zuführung, Sortierung und schwerkraftbedingte Rückführung an der Bühne, sowie für die Inspektion der hierfür notwendigen Anlagenteile und Vorrichtungen, vorzusehen.

Dadurch, dass der Linearantrieb in einer Förderrichtung und einer Gegenförderrichtung betreibbar ist, ist es möglich, Behälterverschlüsse, die sich in der Behandlungskammer befinden, auch entgegen der Förderrichtung wieder aus der Behandlungskammer herausbewegen zu können. Insbesondere können sich bei einem Anlagenstopp in der Kammer befindliche Behälterverschlüsse wieder rückwärts aus dieser herausgeführt werden und nach einem Weiterbetreiben der Anlage wieder in die Behandlungskammer zurückgeführt werden, sodass die unterbrochene Behandlung der Behälterverschlüsse fortgesetzt beziehungsweise erneut begonnen werden kann.

Dadurch, dass die Behälterverschlüsse rückwärts aus der Behandlungskammer herausgeführt werden, sind der Behandlungskammer nachgelagert keine zusätzlichen Strecken oder Pufferstrecken vorzusehen. Mithin kann der Verschließer zum Verschließen von Behältern mit den Behälterverschlüssen der Behandlungskammer direkt nachgelagert angeordnet sein. Dadurch wird ein besonders kompakter Aufbau einer die Vorrichtung aufweisenden Anlage erzielt.

Bei einem Anlagenstopp einer die Vorrichtung zum Behandeln von Behälterverschlüssen umfassenden Anlage, beispielsweise einer Getränkeabfüllanlage, können in der Behandlungskammer befindliche Behälterverschlüsse damit durch den Linearantrieb aktiv aus der Behandlungskammer heraus bewegt werden, so dass eine Überbehandlung beziehungsweise eine Beschädigung der Behälterverschlüsse vermieden werden kann. Bevorzugt können die während des Anlagenstopps aus der Behandlungskammer herausbewegten Behälterverschlüsse nach einem erneuten Anlaufen der Anlage der Behandlungskammer erneut der Behandlung zugeführt werden. Folglich lässt sich der Ausschuss von Behälterverschlüssen bei einem Anlagenstopp signifikant verringern.

Durch den aktiven Transport der Behälterverschlüsse mittels des Linearantriebs können auch Verformungen der Behälterverschlüsse vermieden werden, da kein Staudruck auf die einzelnen Behälterverschlüsse wirkt und diese vielmehr im Wesentlichen ohne gegenseitige Berührung zueinander geführt werden.

Fernerhin kann durch den Linearantrieb ein nachgelagerter Verschließer taktgenau mit Behälterverschlüssen versorgt werden. Ein zusätzlicher Stopper zwischen der Transportvorrichtung und dem Verschließer, beziehungsweise einer Pick&Place Station oder einem Positioniersystem des Verschließers ist somit nicht erforderlich.

Ein Einschleusen und Ausschleusen von Behälterverschlüssen in die Behandlungskammer hinein beziehungsweise aus der Behandlungskammer heraus kann durch den Linearantrieb zudem besonders effizient gestaltet werden. Insbesondere, wenn die Behandlungskammer eine mehrtürige Schleuse zum Einschleusen und/oder Ausschleusen aufweist, kann ein durch eine erste Tür der Schleuse eingeschleuster Behälterverschluss in der Schleuse abgestoppt beziehungsweise verlangsamt werden, bis die erste Tür wieder verschlossen und die zweite Tür anschließend geöffnet wurde. Entsprechend kann auch bei einem Ausschleusen eines Behälterverschlusses aus der Behandlungskammer verfahren werden. Damit kann weiterhin aufgrund der verbesserten Schleusenabdichtung der Einsatz des Desinfektionsmittels verringert werden, da die Verluste an der Schleuse verringert werden.

Folglich kann durch das Versehen der Transportvorrichtung mit einem Linearantrieb eine Vorrichtung zum Behandeln eines Behälterverschlusses mit einem besonders kompakten, effizienten und einfachen Aufbau bereitgestellt werden.

Erfindungsgemäß weist die Transportvorrichtung einen Langstator und mindestens einen über den Langstator individuell antreibbaren Läufer zum Aufnehmen mindestens eines zu behandelnden Behälterverschlusses auf, wobei bevorzugt eine Mehrzahl von Läufern vorgesehen ist. Weiterhin ist es möglich, dass ein Läufer zum Aufnehmen einer Mehrzahl von Behälterverschlüssen ausgebildet ist.

Der Linearantrieb ist in Form eines Linearmotors ausgebildet, wobei ein Stator in Form eines Langstators vorgesehen ist, mittels welchem der Transportweg beschrieben wird, und die Läufer auf diesem Langstator individuell bewegt werden.

Durch den individuell antreibbaren Läufer kann entsprechend die Position sowie die Bewegung eines Behälterverschlusses oder mehrerer Behälterverschlüsse in und durch die Behandlungskammer individuell gesteuert beziehungsweise eingestellt werden. Zudem ist dadurch ein taktgenaues Aufnehmen eines Behälterverschlusses von einer Zuführung sowie ein taktgenaues Übergeben eines Behälterverschlusses an den Verschließer möglich. Der Langstator stellt dabei bevorzugt eine Bewegungsbahn des Läufers beziehungsweise einen Kreislauf beziehungsweise eine Haupttransportlinie für den Läufer bereit.

Mit jedem Läufer kann auch mehr als ein Behälterverschluss transportiert werden. Auf diese Weise lässt sich ein effizientes Transportieren der Behälterverschlüsse erreichen bei einer reduzierten Anzahl von Läufern.

Gemäß einer weiteren bevorzugten Ausführungsform kann der Langstator auch außerhalb einer Wand der Behandlungskammer angeordnet sein und der Läufer kann über eine Führung auf der anderen Seite der Wand in der Behandlungskammer geführt sein. Dadurch wird vermieden, dass der Langstator mit dem Behandlungsmedium in der Behandlungskammer in Kontakt kommt.

Wenn ein Läufer gemäß einer weiteren bevorzugten Ausführungsform einen Mitnehmer zum Mitnehmen eines Behälterverschlusses aus einer Zuführrinne aufweist, kann der Läufer ferner als Stopper an der Zuführrinne fungieren. Bevorzugt wird bei einer Übernahme der Behälterverschlüsse aus der Zuführrinne, beispielsweise einer Zuführrinne eines Sortierwerks, stets ein Läufer am Ausgang der Zuführrinne positioniert, sodass ein Herausfallen eines Behälterverschlusses aus der Zuführrinne verhindert ist. Der Mitnehmer sorgt dabei dafür, dass ein aus der Zuführrinne auf einen den Mitnehmer aufweisenden Läufer übergebener Behälterverschluss bei einem Wegbewegen des Läufers von der Zuführrinne zuverlässig von dem Läufer mitgenommen werden kann. Zudem kann der Mitnehmer eines nächsten Läufers, welcher nachdem ein vorheriger Läufer von der Zuführrinne wegbewegt wurde, an dessen Position bewegt wird, einen weiteren Behälterverschluss aufnehmen und bevorzugt mittels seines Mitnehmers verhindern, dass weitere Behälterverschlüsse aus der Zuführrinne heraus gleiten beziehungsweise heraustreten können.

Auch wenn die Transportvorrichtung einen Puffer zum Zwischenspeichern von Behälterverschlüssen aufweist, können Behälterverschlüsse, die etwa während eines Anlagenstopps aus der Behandlungskammer herausgeführt werden, zwischengespeichert werden. Zudem ist es dadurch möglich, bei Störungen von Teilen der Anlage oder der Vorrichtung, beispielsweise bei Störungen der Zuführung von Behälterverschlüssen an die Transportvorrichtung, die Anlage ohne nennenswerte Unterbrechung weiter zu betreiben.

Gemäß einer weiteren bevorzugten Ausführungsform weist die Transportvorrichtung eine Weiche auf, wobei der Puffer über die Weiche mit einer Haupttransportlinie der Transportvorrichtung verbunden ist, wobei bevorzugt eine erste Weiche zum Anbinden des Puffers an einer ersten Stelle der Haupttransportlinie und eine zweite Weiche zum Anbinden des Puffers an einer zweiten Stelle der Haupttransportlinie vorgesehen ist. Dadurch ist es möglich, aus der Behandlungskammer herausgeleitete Behälterverschlüsse über die Weiche, bevorzugt die erste Weiche, in den Puffer zu bewegen, und nach einem Zwischenspeichern wieder über eine Weiche, entweder der ersten Weiche oder der zweiten Weiche, der Haupttransportlinie zurückzuführen. Zudem wird durch die zweite Weiche ermöglicht, dass auch die Haupttransportlinie, welche einen regulären Kreislauf ausbildet, zumindest teilweise als zusätzliche Pufferstrecke genutzt werden kann.

Wenn die Transportvorrichtung dazu ausgebildet ist, den Behälterverschluss direkt einem Verschließer zum Verschließen eines Behälters mit einem Behälterverschluss zuzuführen, kann ein besonders einfacher Aufbau einer die Vorrichtung aufweisenden Anlage, insbesondere einer die Vorrichtung aufweisenden Getränkeabfüllanlage, bereitgestellt werden.

Gemäß einer weiteren bevorzugten Ausführungsform ist die Behandlungskammer als Sterilisationskammer für Behälter, bevorzugt für Preforms der Behälter, und ferner für die Behälterverschlüsse zum Verschließen der Behälter, ausgebildet. Dadurch ist es möglich, sowohl die Behälter, bevorzugt die Preforms der Behälter, als auch die Behälterverschlüsse in einer gemeinsamen Behandlungskammer zu behandeln. Dies wird ermöglicht, da durch den Linearantrieb zum einen eine bodennahe Behandlung möglich ist und zum anderen aufgrund der individuell steuerbaren Positionierung beziehungsweise Geschwindigkeit der Bewegung der zu behandelnden Behälterverschlüsse in der Behandlungskammer die Verweilzeit beziehungsweise Behandlungszeit der Behälterverschlüsse und der Behälter in der Behandlungskammer individuell und unabhängig voneinander einstellbar ist.

Mit anderen Worten ist eine Verweilzeit beziehungsweise Behandlungszeit der Behälterverschlüsse weitgehend unabhängig von der Verweilzeit beziehungsweise Behandlungszeit der Behälter beziehungsweise Preforms in der gleichen Behandlungskammer. Folglich kann eine anderweitig vorzusehende weitere Behandlungskammer für die Behälterverschlüsse oder die Behälter entfallen, und damit auch ein zweiter Verdampfer für das Sterilisationsmedium, eine zweite Vorrichtung zur Luftkonditionierung mit Wärmetauscher und eine Regelung dieser zweiten Behandlungskammer sowie eine Überwachungsvorrichtung für die vorgenannten Komponenten. Eine Anlage, welche die vorgeschlagene Vorrichtung aufweist, kann entsprechend im Vergleich zu herkömmlichen Anlagen kompakter und einfacher aufgebaut sein. Zudem ist deren Prozesssicherheit erhöht. Ebenso benötigt eine derart ausgebildete Vorrichtung eine geringere notwendige maximale Bauhöhe im Vergleich zu herkömmlichen Vorrichtungen zum Behandeln von Behälterverschlüssen.

In einer bevorzugten weiteren Ausführungsform sind die einzelnen Läufer einer Mehrzahl von Läufern individuell ansteuerbar. Mithin können die Geschwindigkeiten der einzelnen Läufer entlang ihres Weges individuell eingestellt und gesteuert werden. Damit können beispielsweise die Läufer mit Verschlüssen, die in der Behandlungskammer schon über die Hälfte der Behandlungsstrecke zurückgelegt haben, bei einem Anlagenstopp mit höherer Geschwindigkeit rückwärtsgefahren werden, um die Verschlüsse keiner zeitlichen Überbehandlung auf der Rückwärtsfahrt auszusetzen. Läuft die Anlage weiter, werden die zurückgefahrenen Verschlüsse nochmal unter den idealen Bedingungen, insbesondere in der dann noch erforderlichen beziehungsweise vorgesehenen Restzeit, durch die Behandlungsstrecke gefahren.

Bevorzugt werden die Läufer auf einem Rückweg zwischen Verschließer und einer Verschlusszufuhr zum Zuführen der jeweils neuen zu behandelnden Verschlüsse zu einem Läufer mit hoher bis sehr hoher Geschwindigkeit zurückbewegt, insbesondere im Vergleich zu ihrer jeweiligen Geschwindigkeit zwischen der Verschlusszufuhr und der Behandlungskammer sowie in der Behandlungskammer. Damit legen sie die Strecke schnell zurück und es können auf diese Weise weniger Läufer auf der gesamten Linie bei gleicher Behandlungsleistung eingesetzt werden.

Um die Behälterverschlüsse vor dem Zuführen in die Behandlungskammer zu reinigen, insbesondere von grobem Schmutz zu befreien, kann der Behandlungskammer vorgelagert eine Reinigungsvorrichtung zum Befreien eines zu behandelnden Behälterverschlusses von Schmutz angeordnet sein. Die Reinigungsvorrichtung kann dabei bevorzugt als Spülvorrichtung oder Blasvorrichtung ausgebildet sein, welche ein flüssiges Spülmedium oder ein Gas, bevorzugt Reinluft, zum Reinigen beziehungsweise Vorreinigen der Behälterverschlüsse verwendet.

Wenn die Transportvorrichtung in Bezug auf ihre Hauptbewegungsrichtung einen gewundenen Abschnitt aufweist, welche derart ausgebildet ist, dass ein sich im gewundenen Abschnitt von der Transportvorrichtung transportierter Behälterverschluss eine Änderung seiner Orientierung erfährt, wobei der gewundene Abschnitt bevorzugt im Bereich der Reinigungsvorrichtung angeordnet ist, kann insbesondere eine Vorreinigung von Behälterverschlüssen vor dem Zuführen in die Behandlungskammer nochmals effektiver gestaltet werden. Bevorzugt ist im gewundenen Abschnitt ein Behälterverschluss zumindest teilweise in dem oder an dem Läufer gehalten.

Bevorzugt sind die Behälterverschlüsse dabei stromaufwärts beziehungsweise dem gewundenen Abschnitt vorgelagert auf der Transportvorrichtung liegend orientiert und erfahren im gewundenen Abschnitt eine Veränderung ihrer Orientierung und bevorzugt ein Kippen beziehungsweise Schwenken, so dass sie neben oder unterhalb der Transportvorrichtung vorliegen. Bevorzugt ist die Reinigungsvorrichtung dabei ebenfalls unterhalb der Transportvorrichtung angeordnet, sodass das Reinigungsmedium der Reinigungsvorrichtung, bevorzugt das Reinigungsfluid, Gas oder Gasgemisch, von unten an den zu reinigenden Behälterverschluss gesprüht beziehungsweise geblasen werden kann. Ferner wird hierdurch ermöglicht, dass sich grobe Schmutzpartikel schwerkraftbedingt entweder bereits vor dem Reinigen oder zumindest nach dem Reinigen in Richtung der Schwerkraft von dem Behälterverschluss lösen.

### Kurze Beschreibung der Figuren

Bevorzugte weitere Ausführungsformen der Erfindung werden durch die nachfolgende Beschreibung der Figuren näher erläutert. Dabei zeigen:
- Figur 1: schematisch eine Vorrichtung zum Behandeln von Behälterverschlüssen;
- Figur 2: schematisch die Vorrichtung aus Figur 1 in einem Regelbetrieb;
- Figur 3: schematisch die Vorrichtung aus Figur 1 kurz nach einem Anlagenstopp der Getränkeabfüllanlage;
- Figur 4: schematisch eine Vorrichtung zum Behandeln von Behälterverschlüssen gemäß einer weiteren Ausführungsform; und
- Figur 5: schematisch eine Vorrichtung zum Behandeln von Behälterverschlüssen gemäß einer nochmals weiteren Ausführungsform.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

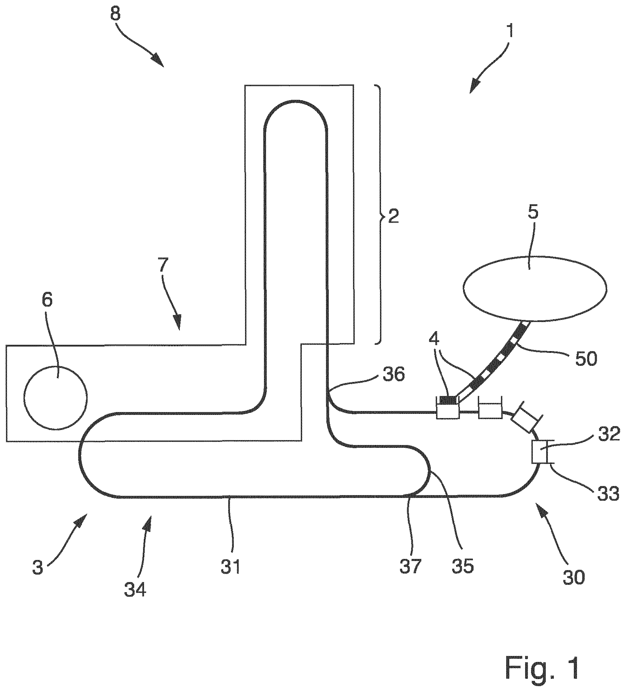

In Figur 1 ist schematisch eine Vorrichtung 1 zum Behandeln von Behälterverschlüssen 4 gezeigt. Die Vorrichtung 1 ist in eine Getränkeabfüllanlage 8 integriert. Die Getränkeabfüllanlage 8 weist auch eine Sortiervorrichtung 5 zum Sortieren von Behälterverschlüssen 4, sowie einen Isolator 7 und einen in dem Isolator 7 befindlichen Verschließer 6 zum Verschließen von Behältern mit den Behälterverschlüssen 4 auf.

Die Sortiervorrichtung 5, der Verschließer 6 sowie der Isolator 7 können auch als Teil der Vorrichtung 1 ausgebildet sein.

Die Vorrichtung 1 weist eine Behandlungskammer 2 auf, welche vorliegend zum Sterilisieren der Behälterverschlüsse 4 ausgebildet ist. Die Behandlungskammer 2 ist vorliegend dem Isolator 7 der Getränkeabfüllanlage 8 zugeordnet. Alternativ kann die Behandlungskammer 2 auch separat zu dem Isolator 7 bereitgestellt sein. Zudem weist sie eine Transportvorrichtung 3 auf, welche einen Linearantrieb 30 umfasst. Der Linearantrieb 30 weist einen Langstator 31 und eine Vielzahl von über den Langstator 31 jeweils individuell antreibbare Läufer 32 zum Aufnehmen von zu behandelnden Behälterverschlüssen 4 auf. Der Linearantrieb 30 ist entsprechend in Form eines Linearmotors aufgebaut, mit einen Langstator 31 und einer Vielzahl von sich auf diesem bewegenden und individuell antreibbaren Läufern 32.

Jeder Läufer 32 weist einen Mitnehmer 33 zum Mitnehmen eines zu transportierenden Behälterverschlusses 4 aus einer Zuführrinne 50 der Sortiervorrichtung 5 auf. Der Läufer 32 und der Mitnehmer 33 können auch so ausgebildet sein, dass jeweils mehr als ein einzelner Behälterverschluss 4 von dem Läufer 32 mitgenommen und transportiert wird. Beispielsweise können auch zwei oder mehr, beispielsweise 4, 6 oder 8 Behälterverschlüsse 4 auf dem Läufer 32 transportiert werden.

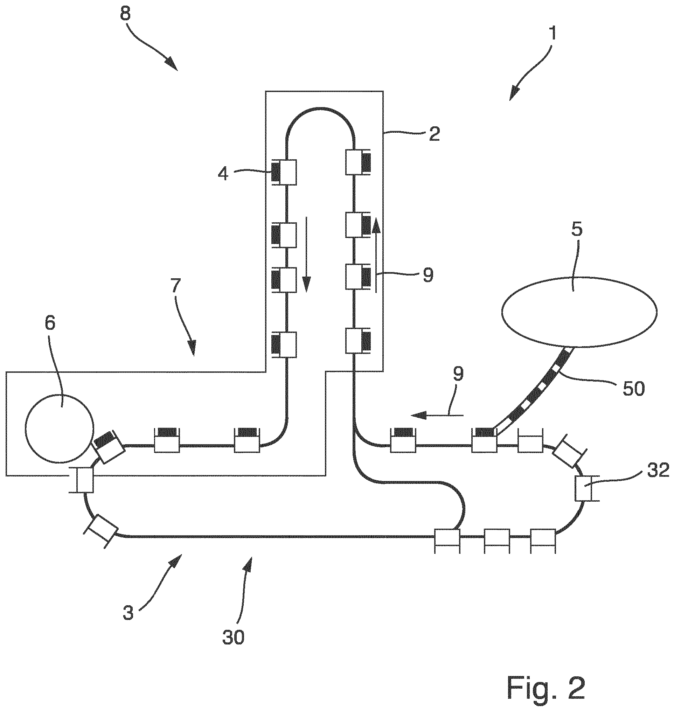

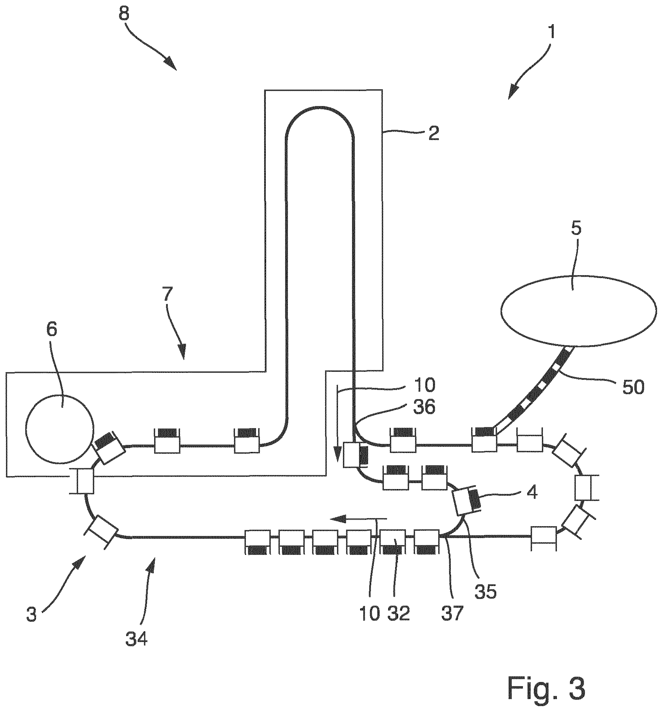

Der Linearantrieb 30 ist vorliegend derart ausgebildet, dass er in einer Förderrichtung (siehe Figur 2) und in einer Gegenförderrichtung (siehe Figur 3) betreibbar ist. Mit anderen Worten können die Läufer 32 in zwei Richtungen entlang der Transportvorrichtung 3 bewegt werden.

Ferner weist die Transportvorrichtung 3 einen Puffer 35 auf, welcher zum Zwischenspeichern von Behälterverschlüssen 4 vorgesehen ist. Der Puffer 35 ist mit einer Haupttransportlinie 34 der Transportvorrichtung 3 über eine erste Weiche 36 und eine zweite Weiche 37 verbunden. Die erste Weiche 36 ist dabei an der Haupttransportlinie 34 zwischen der Zuführrinne 50 und der Behandlungskammer 2 angeordnet, die zweite Weiche 37 ist dem Verschließer 6 und dem Isolator 7 nachgelagert an der Transportvorrichtung 3 angeordnet.

Figur 2 zeigt schematisch die Vorrichtung 1 aus Figur 1 in einem Regelbetrieb, in welchem die Transportvorrichtung 3 die Läufer 32 in der Förderrichtung 9 individuell bewegt. Aus der Sortiervorrichtung 5 über die Zuführrinne 50 an die Transportvorrichtung 3 zugeführte Behälterverschlüsse 4 werden an dem Ende der Zuführrinne 50 einzeln von jeweils einem Läufer 32 mitgenommen. Nach dem Übernehmen eines Behälterverschlusses 4 durch einen Läufer 32 am Ende der Zuführrinne 50 wird der den Behälterverschluss 4 tragende Läufer 32 in die Behandlungskammer 2 geschleust. In der Behandlungskammer 2 wird der Behälterverschluss 4 mit einem dampfförmigen Behandlungsmedium, vorliegend verdampftes Wasserstoffperoxid, beaufschlagt. Alternativ kann auch ein anderes an sich bekanntes Behandlungsmedium, beispielsweise Peressigsäure, in der Behandlungskammer 2 verwendet werden, um die Behandlung der Behälterverschlüsse zu erzielen.

Die Verweilzeit beziehungsweise Behandlungszeit jedes Behälterverschlusses 4 in der Behandlungskammer 2 wird durch die individuell vorgegebene Geschwindigkeit des Läufers 32 vorgegeben und kann entsprechend genau eingestellt werden. Eine Überbehandlung oder ungenügende Behandlung kann somit effektiv vermieden werden. Nach dem Ausschleusen der behandelten Behälterverschlüsse 4 aus der Behandlungskammer 2 werden diese im Isolator 7 weiter geführt und direkt und taktgenau an den Verschließer 6 übergeben. Nach dem Übergeben des Behälterverschlusses 4 an den Verschließer 6 wird der Läufer 32 wieder aus dem Isolator 7 herausgeschleust und entlang der Transportvorrichtung 3 entlang des Kreislaufs der Haupttransportlinie 34 weiter in Richtung und zur Zuführrinne 50 bewegt, sodass ein neuer zu behandelnder Behälterverschluss 4 aufgenommen werden kann. Auf diesem "Rückweg" kann der jeweilige leere Läufer 32 mit hoher Geschwindigkeit bewegt werden, um auf diese Weise die Anzahl der benötigten Läufer 32 gering zu halten.

Figur 3 zeigt schematisch Vorrichtung 1 aus Figur 1 kurz nach einem Anlagenstopp der Getränkeabfüllanlage 8. Um zu verhindern, dass Behälterverschlüsse 4, welche sich als der Anlagenstopp auftrat bereits in der Behandlungskammer 2 befanden, überbehandelt werden, wurden diese in der Gegenrichtung 10 entgegen der normalen Förderrichtung 9 (siehe Figur 2) aus der Behandlungskammer 2 zurück bewegt. Dabei werden diejenigen Läufer 32, welche Verschlüsse tragen, die schon zu mehr als 50% behandelt wurden, besonders schnell aus der Behandlungskammer 2 herausgefahren.

Die Läufer 32 gelangen dabei über die erste Weiche 36 auf beziehungsweise in den Puffer 35. Da sich mehr Läufer 32 in der Behandlungskammer 2 befanden, als der Puffer 35 aufnehmen kann, wurde ein Teil der Läufer 32, welche sich zum Anlagenstopp in der Behandlungskammer 2 befanden, über die zweite Weiche 37 weiter auf die Haupttransportlinie 34 gefördert. Mit anderen Worten dient der Bereich der Haupttransportlinie 34, welcher zum Rückführen der Läufer 34 nach dem Ausschleusen aus dem Isolator 7 vorgesehen ist, als ein weiterer Puffer beziehungsweise als Verlängerung des Puffers 35.

Nach Beendigung des Anlagenstopps werden die im Puffer 35 und im weiteren Puffer befindlichen Läufer 32 wieder in Förderrichtung 9 zu der und in die Behandlungskammer 2 gefördert, sodass die Behälterverschlüsse 4 eine ordnungsgemäße Sterilisierung erfahren. Nachdem der Puffer 35 sowie der weitere Puffer entleert sind, werden wieder neue Behälterverschlüsse 4 von der Zuführrinne 50 aufgenommen und wie in Figur 2 gezeigt, folglich im Normalbetrieb, durch die Behandlungskammer 2 bewegt.

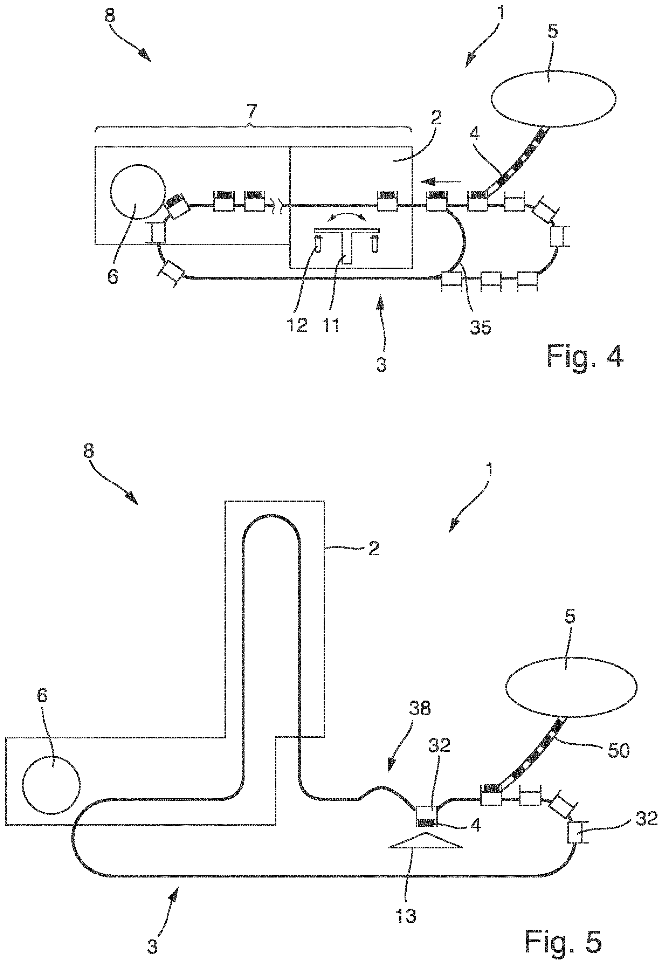

Figur 4 zeigt schematisch eine Vorrichtung 1 zum Behandeln von Behälterverschlüssen 4 gemäß einer weiteren Ausführungsform. Sie entspricht im Wesentlichen der Vorrichtung aus Figur 1, wobei die Behandlungskammer 2 nicht nur zum Behandeln der Behälterverschlüsse 4 vorgesehen ist, sondern vorliegend als Sterilisationskammer für Preforms 12 der Behälter ausgebildet ist, welche nach dem Formblasen und Befüllen mit einem Füllprodukt im Verschließer 6 durch die Behälterverschlüsse 4 verschlossen werden sollen. Die Behandlungskammer 2 ist ferner zum Behandeln der Behälterverschlüsse 4 zum Verschließen der Behälter ausgebildet. Mithin erfolgt eine Sterilisierung sowohl der Behälterverschlüsse 4, welche mittels der Transportvorrichtung 3 durch die Behandlungskammer 2 bewegt werden, als auch der Preforms 12, welche durch eine separat zu der Transportvorrichtung 3 bereitgestellte Preformtransportvorrichtung 11 ebenfalls durch die Behandlungskammer 2 transportiert werden. Die Behandlungszeiten der Behälterverschlüsse 4 und der Preforms 12 ist dabei unterschiedlich und an die jeweiligen Formstücke angepasst. Dadurch ist es möglich, dass sowohl die Preforms 12 als auch die Behälterverschlüsse 4 eine Behandlung mit einer optimalen Behandlungszeit in der Behandlungskammer 2 erfahren.

Figur 5 zeigt schematisch eine Vorrichtung 1 zum Behandeln von Behälterverschlüssen 4 gemäß einer weiteren Ausführungsform. Sie entspricht im Wesentlichen der in Figur 1 gezeigten Vorrichtung, wobei in dieser Ausführungsform kein Puffer vorgesehen ist. Alternativ kann jedoch auch die in Figur 5 gezeigte Vorrichtung 1 einen Puffer gemäß den zuvor beschriebenen Ausführungsformen umfassen. Zwischen der Zuführrinne 50 und der Behandlungskammer 2 weist die Transportvorrichtung 3 einen gewundenen Abschnitt 38 auf. In diesem gewundenen Abschnitt 38 erfahren die Läufer 32 jeweils eine Änderung ihrer Orientierung. Während die Läufer 32 am Ende der Zuführrinne 50 mit ihrer Aufnahme für den Behälterverschluss 4 nach oben, entsprechend entgegen der Schwerkraft, weisen, wird die Orientierung der Läufer 32 im gewundenen Abschnitt 38 derart geschwenkt, sodass die Läufer 32 mit ihren Aufnahmen jeweils nach unten, entsprechend in Richtung der Schwerkraft weisen. Mithin weist ein in dem geschwenkten Läufer 32 gehaltener beziehungsweise aufgenommener Behälterverschluss 4 ebenfalls nach unten. Unterhalb des gewundenen Abschnitts 38 ist eine Reinigungsvorrichtung 13 angeordnet, welche die Behälterverschlüsse 4 vor dem Einschleusen in die Behandlungskammer 2 vorliegend durch Ausblasen mit Druckluft von grobem Schmutz und Staub befreit. Nachdem der Behälterverschluss 4 durch die Reinigungsvorrichtung 13 vorgereinigt wurde, wird der diesen Behälterverschluss 4 aufweisende Läufer 32 im restlichen Teil des gewundenen Abschnitt 38 weiter geschwenkt, sodass er wieder seine ursprüngliche Ausrichtung aufweist.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

### Bezuqszeichenliste

- 1: Vorrichtung
- 2: Behandlungskammer
- 3: Transportvorrichtung
- 30: Linearantrieb
- 31: Langstator
- 32: Läufer
- 33: Mitnehmer
- 34: Haupttransportlinie
- 35: Puffer
- 36: Erste Weiche
- 37: Zweite Weiche
- 38: Gewundener Abschnitt
- 4: Behälterverschluss
- 5: Sortiervorrichtung
- 50: Zuführrinne
- 6: Verschließer
- 7: Isolator
- 8: Getränkeabfüllanlage
- 9: Förderrichtung
- 10: Gegenrichtung
- 11: Preformtransportvorrichtung
- 12: Preform
- 13: Reinigungsvorrichtung

## Patentansprüche

1. Vorrichtung (1) zum Behandeln von Behälterverschlüssen (4), bevorzugt zum Sterilisieren von Behälterverschlüssen (4) zum Verschließen von Behältern in einer Getränkeabfüllanlage (8), umfassend eine Behandlungskammer (2) zum Behandeln von Behälterverschlüssen (4), und eine Transportvorrichtung (3) zum Transportieren von Behälterverschlüssen (4) durch die Behandlungskammer (2), wobei
die Transportvorrichtung (3) einen Linearantrieb (30) umfasst, der einen Langstator (31) und zumindest einen über den Langstator (31) individuell antreibbaren Läufer (32) zum Aufnehmen eines zu behandelnden Behälterverschlusses (4) oder eine Mehrzahl von zu behandelnden Behälterverschlüssen (4) aufweist,
**dadurch gekennzeichnet, dass**
der Linearantrieb (30) in einer Förderrichtung (9) und einer Gegenförderrichtung (10) betreibbar ist, so dass Behälterverschlüsse (4), die sich in der Behandlungskammer (2) befinden, entgegen der Förderrichtung (9) aus der Behandlungskammer herausbewegbar sind.

2. Vorrichtung (1) gemäß Anspruch 1, **dadurch gekennzeichnet, dass** eine Mehrzahl von Läufern (32) vorgesehen ist.

3. Vorrichtung (1) gemäß Anspruch 2, **dadurch gekennzeichnet, dass** ein Läufer (32) einen Mitnehmer (33) zum Mitnehmen eines Behälterverschlusses (4) aus einer Zuführrinne (50) aufweist.

4. Vorrichtung gemäß Anspruch 2 oder 3, **dadurch gekennzeichnet, dass** mit jedem Läufer (32) mehr als ein Behälterverschluss (4) transportiert werden kann.

5. Vorrichtung (1) gemäß einem der vorstehenden Ansprüche, **dadurch gekennzeichnet, dass** die Transportvorrichtung (3) einen Puffer (35) zum Zwischenspeichern von Behälterverschlüssen (4) aufweist.

6. Vorrichtung (1) gemäß Anspruch 5, **dadurch gekennzeichnet, dass** die Transportvorrichtung (3) eine Weiche (36, 37) aufweist, wobei der Puffer (35) über die Weiche (36, 37) mit einer Haupttransportlinie (34) der Transportvorrichtung (3) verbunden ist, wobei bevorzugt eine erste Weiche (36) zum Anbinden des Puffers (35) an einer ersten Stelle der Haupttransportlinie (34) und eine zweite Weiche (37) zum Anbinden des Puffers (35) an einer zweiten Stelle der Haupttransportlinie (34) vorgesehen ist.

7. Vorrichtung (1) gemäß einem der vorstehenden Ansprüche, **dadurch gekennzeichnet, dass** die Transportvorrichtung (3) dazu ausgebildet ist, den Behälterverschluss (4) direkt einem Verschließer (6) zum Verschließen eines Behälters mit dem Behälterverschluss (4) zuzuführen.

8. Vorrichtung (1) gemäß einem der vorstehenden Ansprüche, **dadurch gekennzeichnet, dass** die Behandlungskammer (2) als Sterilisationskammer für Behälter, bevorzugt für Preforms (12) der Behälter, und ferner für die Behälterverschlüsse (4) zum Verschließen der Behälter ausgebildet ist.

9. Vorrichtung (1) gemäß einem der vorstehenden Ansprüche, **dadurch gekennzeichnet, dass** der Behandlungskammer (2) vorgelagert eine Reinigungsvorrichtung (13) zum Reinigen eines zu behandelnden Behälterverschlusses (4) angeordnet ist.

10. Vorrichtung (1) gemäß einem der vorstehenden Ansprüche, **dadurch gekennzeichnet, dass** die Transportvorrichtung (3) in Bezug auf ihre Hauptbewegungsrichtung einen gewundenen Abschnitt (38) aufweist, welcher derart ausgebildet ist, dass ein sich im gewundenen Abschnitt von der Transportvorrichtung (3) transportierter Behälterverschluss (4) eine Änderung seiner Orientierung erfährt, wobei der gewundene Abschnitt (38) bevorzugt im Bereich der Reinigungsvorrichtung (13) angeordnet ist.

## Claims

1. Device (1) for treating container closures (4), preferably for sterilising container closures (4) for closing containers in a beverage filling plant (8), comprising a treatment chamber (2) for treating container closures (4), and a transport device (3) for transporting container closures (4) through the treatment chamber (2), wherein
the transport device (3) comprises a linear drive (30) which has an elongate stator (31) and, for receiving a container closure (4) that is to be treated or a plurality of container closures (4) that are to be treated, at least one carriage (32) that can be driven individually via the elongate stator (31),
**characterised in that**
the linear drive (30) can be operated in a conveying direction (9) and a counter-conveying direction (10), so that container closures (4) located in the treatment chamber (2) can be moved out of the treatment chamber in a direction contrary to the conveying direction (9).

2. Device (1) according to claim 1, **characterised in that** a plurality of carriages (32) is provided.

3. Device (1) according to claim 2, **characterised in that** a carriage (32) has a carrier (33) for carrying a container closure (4) from a feed chute (50).

4. Device according to claim 2 or 3, **characterised in that** more than one container closure (4) can be transported with each carriage (32).

5. Device (1) according to any one of the preceding claims, **characterised in that** the transport device (3) comprises a buffer (35) for intermediate storage of container closures (4).

6. Device (1) according to claim 5, **characterised in that** the transport device (3) has a switch (36, 37), the buffer (35) being connected to a main transport line (34) of the transport device (3) via the switch (36, 37), wherein preferably a first switch (36) is provided for connecting the buffer (35) at a first point of the main transport line (34) and a second switch (37) is provided for connecting the buffer (35) at a second point of the main transport line (34).

7. Device (1) according to any one of the preceding claims, **characterised in that** the transport device (3) is configured to feed the container closure (4) directly to a capping machine (6) for closing a container with the container closure (4).

8. Device (1) according to any one of the preceding claims, **characterised in that** the treatment chamber (2) is configured as a sterilisation chamber for containers, preferably for preforms (12) of the containers, and further for the container closures (4) for closing the containers.

9. Device (1) according to any one of the preceding claims, **characterised in that** a cleaning device (13) for cleaning a container closure (4) that is to be treated is arranged upstream of the treatment chamber (2).

10. Device (1) according to any one of the preceding claims, **characterised in that** the transport device (3) has, with respect to its main direction of movement, a winding portion (38) which is configured in such a way that a container closure (4) transported by the transport device (3) in the winding portion undergoes a change in its orientation, the winding portion (38) preferably being arranged in the region of the cleaning device (13).

## Revendications

1. Dispositif (1) pour le traitement de fermetures de récipients (4), de préférence pour la stérilisation de fermetures de récipients (4) en vue du scellement de récipients dans un équipement d'embouteillage de boissons (8), comprenant une chambre de traitement (2) pour traiter des fermetures de récipients (4), et un dispositif de transport (3) pour transporter des fermetures de récipients (4) à travers la chambre de traitement (2), dans lequel
le dispositif de transport (3) comprend un entraînement linéaire (30) qui comporte un stator fixe (31) et au moins un rotor (32) pouvant être actionné individuellement via le stator fixe (31) en vue de réceptionner une fermeture de récipient (4) à traiter ou une pluralité de fermetures de récipients (4) à traiter,
**caractérisé en ce que**
l'entraînement linéaire (30) peut fonctionner dans un sens de refoulement (9) et un contre-sens de refoulement (10), de sorte que des fermetures de récipients (4) qui se trouvent dans la chambre de traitement (2) peuvent être déplacées hors de la chambre de traitement à l'encontre du sens de refoulement (9).

2. Dispositif (1) selon la revendication 1, **caractérisé en ce qu'**une pluralité de rotors (32) est prévue.

3. Dispositif (1) selon la revendication 2, **caractérisé en ce qu'**un rotor (32) comporte un entraîneur (33) permettant d'entraîner une fermeture de récipient (4) hors d'un couloir d'alimentation (50).

4. Dispositif (1) selon les revendications 2 ou 3, **caractérisé en ce que** chaque rotor (32) permet de transporter plus d'une fermeture de récipient (4).

5. Dispositif (1) selon l'une quelconque des revendications précédentes, **caractérisé en ce que** le dispositif de transport (3) comporte un circuit tampon (35) pour le stockage intermédiaire des fermetures de récipients (4).

6. Dispositif (1) selon la revendication 5, **caractérisé en ce que** le dispositif de transport (3) comprend une dérivation (36, 37), dans lequel le circuit tampon (35) est relié via la dérivation (36, 37) à une ligne de transport principale (34) du dispositif de transport (3), dans lequel de préférence une première dérivation (36) destinée à relier le circuit tampon (35) à une première position de la ligne de transport principale (34) et une deuxième dérivation (37) destinée à relier le circuit tampon (35) à une deuxième position de la ligne de transport principale (34) sont prévues.

7. Dispositif (1) selon l'une quelconque des revendications précédentes, **caractérisé en ce que** le dispositif de transport (3) est configuré de manière à fournir la fermeture de récipient (4) directement à un dispositif de fermeture (6) pour le scellement d'un récipient avec la fermeture de récipient (4).

8. Dispositif (1) selon l'une quelconque des revendications précédentes, **caractérisé en ce que** la chambre de traitement (2) est conçue sous forme de chambre de stérilisation pour des récipients, de préférence pour des préformes (12) des récipients, et en outre pour les fermetures de récipients (4) pour le scellement des récipients.

9. Dispositif (1) selon l'une quelconque des revendications précédentes, **caractérisé en ce qu'**un dispositif de nettoyage (13) pour le nettoyage d'une fermeture de récipient (4) à traiter est disposé en amont de la chambre de traitement (2).

10. Dispositif (1) selon l'une quelconque des revendications précédentes, **caractérisé en ce que** le dispositif de transport (3) présente par rapport à son sens de déplacement principal un tronçon sinueux (38) qui est conçu de telle sorte qu'une fermeture de récipient (4) transportée dans le tronçon sinueux par le dispositif de transport (3) subit une modification de son orientation, le tronçon sinueux (38) étant disposé de préférence dans la zone du dispositif de nettoyage (13).
